# EUROPEAN PATENT SPECIFICATION

(11) **EP 4 110 439 B1**
(45) Date of publication and mention of the grant of the patent: **24.12.2025**
(21) Application number: 21707626.4
(22) Date of filing: 10.02.2021
(51) Int. Cl.: A61M 5/315

(54) **SYRINGE WITH BRAKE MEANS**
SPRITZE MIT BREMSVORRICHTUNG
SERINGUE DOTÉE DE MOYENS DE FREINAGE

(30) Priority: 27.02.2020 GB 202002791
(43) Date of publication of application: 04.01.2023
(60) Divisional application: 25212878.0
(73) Proprietor: Obrist Closures Switzerland GmbH, 4132 Muttenz (CH)
(72) Inventor: CHAVE, Frederic, 69830 Saint-Georges-de-Reneins (FR); BARDET, Philippe, 69830 Saint-Georges-de-Reneins (FR)
(74) Representative: Bryers Intellectual Property Ltd
(86) International application number: PCT/EP2021/053123
(87) International publication number: WO 2021/170401

(56) References cited:
- WO-A2-2018/183772
- US-A- 2 037 768

## Description

The present invention relates generally to syringe and particularly, although not exclusively, to a syringe of the type comprising a tube/barrel/body and a plunger/piston.

A syringe is a simple reciprocating pump consisting of a plunger/piston that fits tightly within a cylindrical tube (often called a barrel). The plunger can be linearly pulled and pushed along the inside of the tube, allowing the syringe to take in and expel liquid or gas through a discharge orifice at a front end of the tube.

Figure I and 2 illustrate analysis of a syringe 10 conducted by the present inventors. It appears that, with constant pressure, the speed of the piston 20 within the barrel 30 accelerates at the end of the stroke (indicated by box B) compared to the initial part of the stroke (illustrated by box A) due to a different tightening between the piston and body in these areas. This can result in a "spray effect" at the end of the stroke.

The present invention seeks to provide improvements in or relating to syringes.

An aspect of the present invention provides a syringe comprising a barrel having an outlet and a piston movable along the inside of the barrel to take in or expel fluid from the outlet, the syringe comprising brake means for influencing movement of the piston.

The goal of some embodiments is to reduce/eliminate the spray effect at the end of use.

The brake means may be effective to slow insertion of the piston over at least part of the stroke.

The brake means may be effective to slow insertion of the piston over only part of the stroke.

The brake means may slow the piston as it approaches the end of the insertion stroke.

The brake mean may be effective to decelerate insertion of the piston towards the end of the stroke; for example during the final approximately 1%, 5%, 10%, 15%, 20%, 25% or 30% of the stroke.

Brake means may be provided on or by the piston. Alternatively or additionally brake means may be provided on or by the barrel.

Brake means may be provided at or towards an end of the piston distal to the barrel outlet.

The brake means may comprise friction means.

The brake means may comprise one or more friction elements, for example ribs, strips, bumps, pads, stickers, roughened zones, interference zones, higher friction zones or the like.

The friction element/s may, for example, comprise one or more longitudinal elongate (for example generally oval) increased friction zones/areas.

In some embodiments the outlet end of the barrel is generally flat. In other embodiments the barrel may comprises a nozzle.

The open end of the syringe may, for example, be fitted with a hypodermic needle, a nozzle or tubing to direct the flow into and out of the barrel.

The barrel and/or the piston may be generally cylindrical with a generally circular section.

The outlet may be located generally centrally. Alternatively the outlet may be located eccentrically.

A further aspect provides a syringe comprising a body and a sliding plunger, the plunger can be linearly moved inside of the body in a pull stroke and a push stroke to draw in or expel liquid from a discharge orifice the body, in which the syringe comprises a friction zone for increasing interference between the plunger and the body to slow down acceleration of the piston towards the end of the push stroke whereby to avoid a spray effect.

The body may be a cylindrical tube or barrel.

The syringe may comprise a cylindrical friction area that is active in the few last millimetres of the insertion stroke.

The friction area may be made up of cylindrical area on the body and bump/s or cylindrical ring/s on the plunger, the interference between these two surfaces brakes the advance of the piston to compensate the acceleration in end of use.

The friction area may be an integral part of the piston/body, or it may be formed separately. A bi-injection process is allowing different materials to be used, with a higher friction material used for brake element/s. The friction zone may be raised from the surface of the piston and/or barrel, or it may be flush. Alternatively of additionally increased friction may be provided by texture (e.g. roughened) and/or surface formations.

The friction element/s are formed from a different material to the barrel/piston.

A further aspect provides a syringe piston with a brake.

In aspects and embodiments of the present invention the syringe may be a reciprocating pump in which a sliding piston fits tightly within a barrel (cylindrical tube). The barrel may have a terminal flange at one (open) end and a central outlet at the other (flat) end. The piston may also have a corresponding terminal flange.

In some embodiments the measured capacity of the barrel may be 5ml. The brake may, for example, be active during the final (approximately) 1ml of the stroke.

To slow down the acceleration of the piston, a "bumper" / "brake" / "deceleration" zone may be added to the piston and may be effective at the end of the insertion/dispensing stroke. With small interference with the barrel, it may brake the piston without stopping the stroke, and then avoid the "spray effect".

In an aspect of the present invention the brake is provided by a plurality of mutually spaced, elongate oval friction elements that are circumferentially distributed and extend longitudinally along the exterior surface of the piston.

Functionality of the syringe may include or be based on: with the piston withdrawn or partially withdrawn this is before the brake is active; the piston is inserted further and just in advance of the brake zone entering the interior of the barrel; the brake zone enters the interior of the barrel to start increasing interference between the piston and the barrel (this may be immediately or maybe (somewhat/slightly) distal to the flange end of the barrel); the piston is fully inserted; the brake zone is frictionally engaged within the interior of the barrel.

The syringe piston may provide a brake function.

In the absence of a brake there may be an acceleration of the piston in the few last millimetre stroke, which can cause a risk of suffocation. This acceleration may be due to a difference in interference between the barrel and the piston head over the length of the barrel and the position at the very end of injection.

To reduce this phenomenon, the syringe may provide a cylindrical friction area that may be active in the few last millimetre of the stroke. It may be made up of a cylindrical area (which may be generally smooth) on the barrel and bumps (or a cylindrical ring, for example) on the piston. The interference between these two surfaces may brake the advance of the piston to compensate the acceleration in end of use.

Different aspects and embodiments of the invention may be used separately or together.

Further particular and preferred aspects of the present invention are set out in the accompanying independent and dependent claims. Features of the dependent claims may be combined with the features of the independent claims as appropriate, and in combination other than those explicitly set out in the claims. Each aspect can be carried out independently of the other aspects or in combination with one or more of the other aspects.

The present invention will now be more particularly described, by way of example, with reference to the accompanying drawings.

The example embodiments are described in sufficient detail to enable those of ordinary skill in the art to embody and implement the systems and processes herein described. It is important to understand that embodiments can be provided in many alternative forms and should not be construed as limited to the examples set forth herein.

Accordingly, while embodiments can be modified in various ways and take on various alternative forms, specific embodiments thereof are shown in the drawings and described in detail below as examples. There is no intent to limit to the particular forms disclosed. On the contrary, all modifications, equivalents, and alternatives falling within the scope of the appended claims should be included. Elements of the example embodiments are consistently denoted by the same reference numerals throughout the drawings and detailed description where appropriate.

Unless otherwise defined, all terms (including technical and scientific terms) used herein are to be interpreted as is customary in the art. It will be further understood that terms in common usage should also be interpreted as is customary in the relevant art and not in an idealised or overly formal sense unless expressly so defined herein.

In the following description, all orientational terms, such as upper, lower, radially and axially, are used in relation to the drawings and should not be interpreted as limiting on the invention.

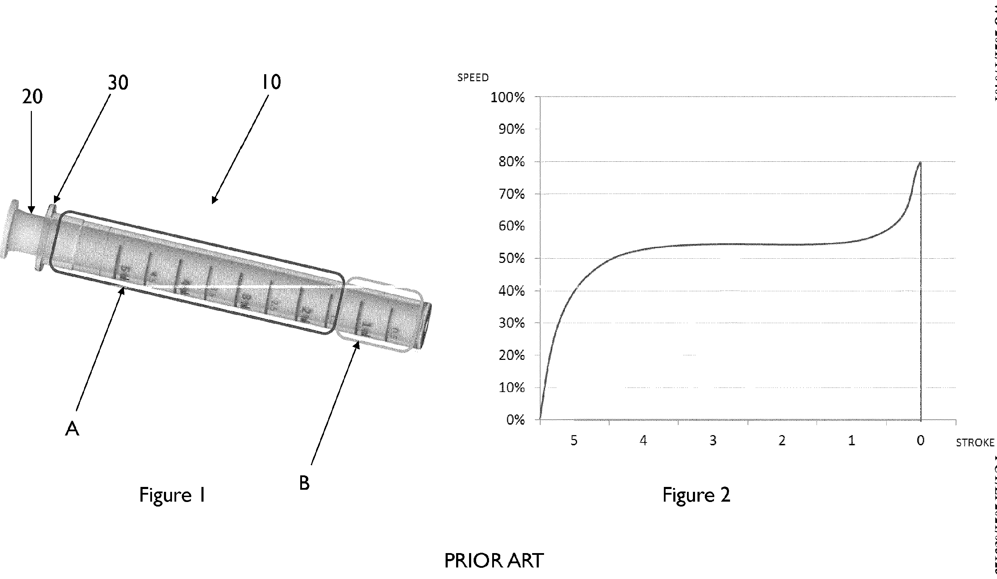

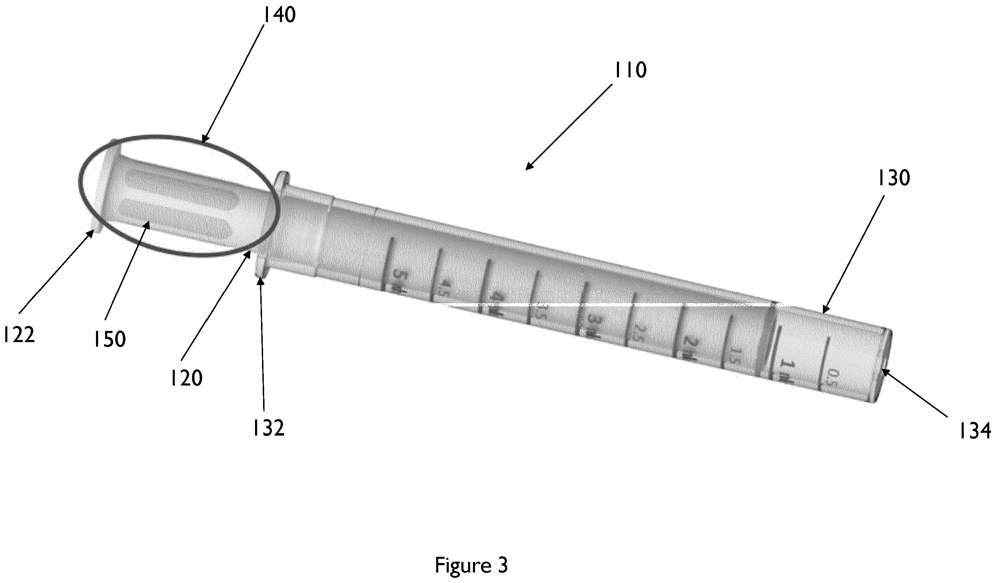

Figure 3 shows a syringe 110 formed in accordance with the present invention.

The syringe is a reciprocating pump in which a sliding piston 120 fits tightly within a barrel 130 (cylindrical tube).

The barrel 130 has a terminal flange 132 at one (open) end and a central outlet 134 at the other (flat) end. The piston 120 also has a corresponding terminal flange 122.

In this embodiment the measured capacity of the barrel is 5ml. The brake is active during the final (approximately) 1ml of the stroke.

To slow down the acceleration of the piston, a "bumper" / "brake" / "deceleration" zone 140 is added to the piston and is effective at the end of the insertion/dispensing stroke. With small interference with the barrel, it will brake the piston without stopping the stroke, and then avoid the "spray effect".

In this embodiment the brake is provided by a plurality of mutually spaced, elongate oval friction elements 150 that are circumferentially distributed and extend longitudinally along the exterior surface of the piston.

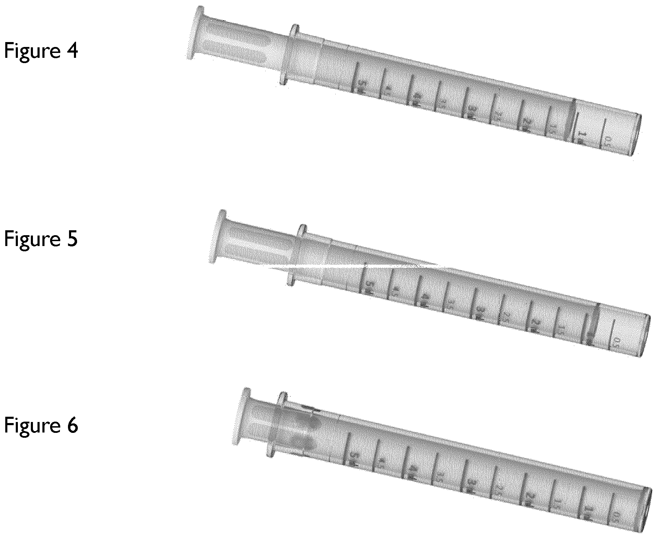

Figures 4 to 6 illustrate the functionality of the syringe 110. Figure 4 shows the piston 120 partially withdrawn and before the brake is active. Figure 5 shows the piston inserted further and just in advance of the brake zone 140 entering the interior of the barrel 130 to start increasing interference between the piston and the barrel. Figure 6 shows the piston fully inserted; the brake zone is shown to be frictionally engaged with the interior of the barrel. Movement from Figure 4 to Figure 5 is unaffected; movement from Figure 5 to Figure 6 is under the influence of increased interference to decelerate the piston.

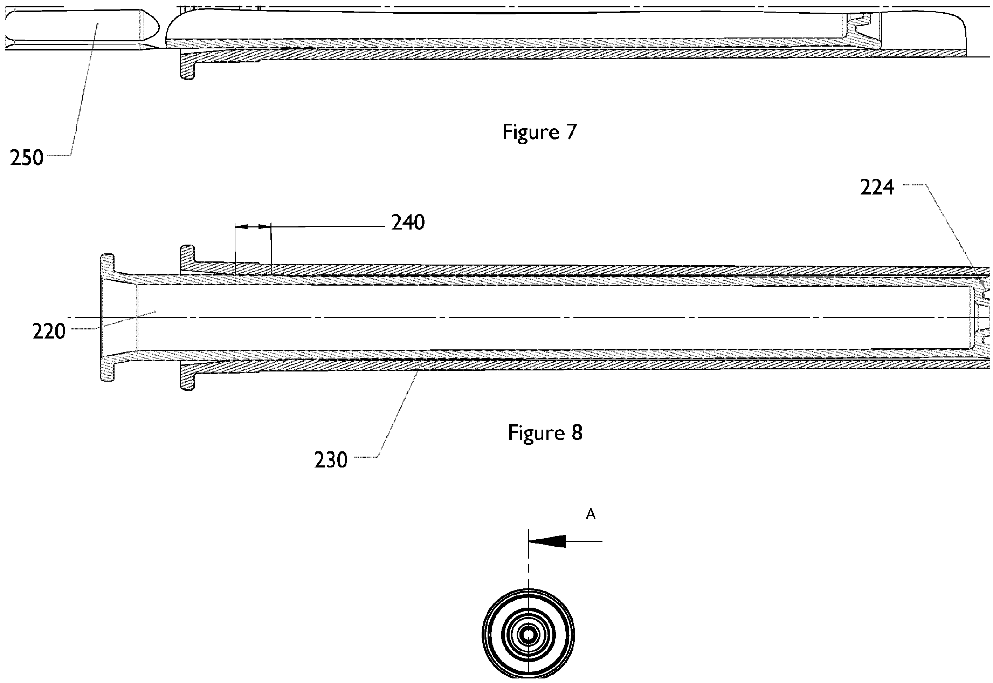

Figures 7 to 9 show a syringe 210 formed in accordance with a further embodiment. The syringe piston provides a brake function.

In the absence of a brake there would be an acceleration of the piston in the few last millimetre stroke, which can cause a risk of suffocation. This acceleration is due to a difference in interference between the barrel and the piston head 224 over the length of the barrel and the position at the very end of injection.

To reduce this phenomenon, the syringe 210 provides a cylindrical friction area 240 that must be active in the few last millimetre of the stroke. It is made up of a cylindrical area (which may be generally smooth) on the barrel and bumps 250 (or a cylindrical ring, for example) on the piston 220. The interference between these two surfaces brakes the advance of the piston to compensate the acceleration in end of use.

Although illustrative embodiments of the invention have been disclosed in detail herein, with reference to the accompanying drawings, it is understood that the invention is not limited to the precise embodiments shown and that various changes and modifications can be effected therein by one skilled in the art without departing from the scope of the invention as defined by the appended claims.

## Claims

1. A syringe comprising a barrel having an outlet and a piston movable along the inside of the barrel to take in or expel fluid from the outlet, the syringe comprising brake means for influencing movement of the piston, in which the brake means is provided at or towards an end of the piston distal to the barrel outlet, **characterised in that** the brake means is provided by a plurality of mutually spaced, elongated and oval-shaped friction elements that are circumferentially distributed and extend longitudinally along an exterior surface of the piston, wherein the friction elements are made from a different material with respect to the material of the piston using a bi-injection process.

2. A syringe as claimed in claim 1, in which the brake means is effective to slow insertion of the piston over at least part of the stroke.

3. A syringe as claimed in claim 1, in which the brake means is effective to slow insertion of the piston over only part of the stroke.

4. A syringe as claimed in claim 3, in which the brake means slows the piston as it approaches the end of the insertion stroke.

5. A syringe as claimed in any preceding claim, in which the brake means is effective to decelerate insertion of the piston towards the end of the stroke.

6. A syringe as claimed in any preceding claim, in which brake means is provided on or by the piston.

7. A syringe as claimed in any preceding claim, in which brake means is provided on or by the barrel.

8. A syringe as claimed in any preceding claim, in which the brake means comprises friction means.

9. A syringe as claimed in any preceding claim, in which the brake means comprises a plurality of friction elements.

10. A syringe as claimed in any preceding claim, in which the friction element/s comprise one or more longitudinal elongate increased friction zones/areas.

11. A syringe as claimed in any preceding claim, in which the outlet end of the barrel is generally flat

12. A syringe as claimed in any of claims 1 to 10, in which the barrel comprises a nozzle

13. A syringe as claimed in any preceding claim, in which the barrel and the piston are generally cylindrical with a generally circular section.

14. A syringe as claimed in any preceding claim, in which the outlet is located generally centrally.

15. A syringe as claimed in any of claims 1 to 13, in which the outlet is located eccentrically.

## Patentansprüche

1. Spritze, umfassend einen Zylinder mit einem Auslass und einen entlang des Inneren des Zylinders bewegbaren Kolben zum Aufnehmen oder Ausstoßen von Fluid aus dem Auslass, wobei die Spritze ein Bremsmittel zum Beeinflussen der Bewegung des Kolbens umfasst, wobei das Bremsmittel an oder zu einem Ende des Kolbens hin distal zu dem Zylinderauslass vorgesehen ist, **dadurch gekennzeichnet, dass** das Bremsmittel durch eine Vielzahl von gegenseitig beabstandeten, länglichen und ovalen Reibelementen bereitgestellt ist, die in Umfangsrichtung verteilt sind und sich in Längsrichtung entlang einer Außenfläche des Kolbens erstrecken, wobei die Reibelemente aus einem anderen Material als dem Material des Kolbens im 2K-Spritzgussverfahren hergestellt sind.

2. Spritze nach Anspruch 1, wobei das Bremsmittel dahingehend wirksam ist, das Einführen des Kolbens über mindestens einen Teil des Hubs zu verlangsamen.

3. Spritze nach Anspruch 1, wobei das Bremsmittel dahingehend wirksam ist, das Einführen des Kolbens über lediglich einen Teil des Hubs zu verlangsamen.

4. Spritze nach Anspruch 3, wobei das Bremsmittel den Kolben verlangsamt, während er sich dem Ende des Einführhubs nähert.

5. Spritze nach einem der vorhergehenden Ansprüche, wobei das Bremsmittel dahingehend wirksam ist, das Einführen des Kolbens zum Ende des Hubs hin zu verlangsamen.

6. Spritze nach einem der vorhergehenden Ansprüche, wobei ein Bremsmittel an dem oder durch den Kolben vorgesehen ist.

7. Spritze nach einem der vorhergehenden Ansprüche, wobei das Bremsmittel an dem oder durch den Zylinder vorgesehen ist.

8. Spritze nach einem der vorhergehenden Ansprüche, wobei das Bremsmittel Reibmittel umfasst.

9. Spritze nach einem der vorhergehenden Ansprüche, wobei das Bremsmittel eine Vielzahl von Reibelementen umfasst.

10. Spritze nach einem der vorhergehenden Ansprüche, wobei das/die Reibelement/e eine/n oder mehrere längliche vergrößerte Längsreibzone/n/-bereich/e umfassen.

11. Spritze nach einem der vorhergehenden Ansprüche, wobei das Auslassende des Zylinders allgemein flach ist.

12. Spritze nach einem der Ansprüche 1 bis 10, wobei der Zylinder eine Düse umfasst.

13. Spritze nach einem der vorhergehenden Ansprüche, wobei der Zylinder und der Kolben allgemein zylindrisch sind und einen allgemein kreisförmigen Querschnitt aufweisen.

14. Spritze nach einem der vorhergehenden Ansprüche, wobei der Auslass allgemein mittig angeordnet ist.

15. Spritze nach einem der Ansprüche 1 bis 13, wobei der Auslass exzentrisch angeordnet ist.

## Revendications

1. Une seringue comprenant un cylindre ayant une sortie et un piston mobile le long de l'intérieur du cylindre pour aspirer ou expulser du fluide de la sortie, la seringue comprenant des moyens de freinage pour influencer le mouvement du piston, dans laquelle les moyens de freinage sont prévus à ou vers une extrémité du piston distale à la sortie du cylindre, **caractérisée en ce que** les moyens de freinage sont fournis par une pluralité d'éléments de friction mutuellement espacés, allongés et de forme ovale qui sont répartis circonférentiellement et s'étendent longitudinalement le long d'une surface extérieure du piston, dans laquelle les éléments de friction sont fabriqués à partir d'un matériau différent par rapport au matériau du piston en utilisant un procédé de bi-injection.

2. Une seringue selon la revendication 1, dans laquelle les moyens de freinage sont efficaces pour ralentir l'insertion du piston sur au moins une partie de la course.

3. Une seringue selon la revendication 1, dans laquelle les moyens de freinage est efficace pour ralentir l'insertion du piston sur une partie seulement de la course.

4. Une seringue selon la revendication 3, dans laquelle les moyens de freinage ralentissent le piston lorsqu'il approche de la fin de la course d'insertion.

5. Une seringue selon l'une quelconque des revendications précédentes, dans laquelle les moyens de freinage sont efficaces pour ralentir l'insertion du piston vers la fin de la course.

6. Une seringue selon l'une quelconque des revendications précédentes, dans laquelle des moyens de freinage sont prévus sur ou par le piston.

7. Une seringue selon l'une quelconque des revendications précédentes, dans laquelle des moyens de freinage sont prévus sur ou par le cylindre.

8. Une seringue selon l'une quelconque des revendications précédentes, dans laquelle les moyens de freinage comprennent des moyens de friction.

9. Une seringue selon l'une quelconque des revendications précédentes, dans laquelle les moyens de freinage comprennent une pluralité d'éléments de friction.

10. Une seringue selon l'une quelconque des revendications précédentes, dans laquelle le ou les éléments de friction comprennent une ou plusieurs zones / surfaces de friction accrues allongées longitudinalement.

11. Une seringue selon l'une quelconque des revendications précédentes, dans laquelle l'extrémité de sortie du cylindre est généralement plate.

12. Une seringue selon l'une quelconque des revendications 1 à 10, dans laquelle le cylindre comprend une buse.

13. Une seringue selon l'une quelconque des revendications précédentes, dans laquelle le cylindre et le piston sont généralement cylindriques avec une section généralement circulaire.

14. Une seringue selon l'une quelconque des revendications précédentes, dans laquelle la sortie est située généralement au centre.

15. Une seringue selon l'une quelconque des revendications 1 à 13, dans laquelle la sortie est située de manière excentrée.
